# EUROPÄISCHE PATENTSCHRIFT

(11) **EP 0 660 737 B1**
(45) Veröffentlichungstag und Bekanntmachung des Hinweises auf die Patenterteilung: **22.07.1998**
(21) Anmeldenummer: 93919019.5
(22) Anmeldetag: 16.09.1993
(51) Int. Cl.: A61N 1/365

(54) **ANORDNUNG ZUR STEUERUNG EINES HERZSCHRITTMACHERS**
ARRANGEMENT FOR CONTROLLING A PACEMAKER
SYSTEME DE COMMANDE D'UN STIMULATEUR CARDIAQUE

(30) Priorität: 17.09.1992 DE 4231601
(43) Veröffentlichungstag der Anmeldung: 05.07.1995
(73) Patentinhaber: BIOTRONIK Mess- und Therapiegeräte GmbH & Co Ingenieurbüro Berlin, D-12359 Berlin (DE)
(72) Erfinder: SCHALDACH, Max, D-91054 Erlangen (DE); BOHEIM, Gustav, D-76887 Wattberg/Zabern (DE)
(74) Vertreter: Christiansen, Henning, Dipl.-Ing.
(86) Internationale Anmeldenummer: DE9300890
(87) Internationale Veröffentlichungsnummer: WO9406513

(56) Entgegenhaltungen:
- US-A- 4 702 253
- US-A- 4 733 667
- BIOMEDIZINISCHE TECHNIK, Bd.32, September 1987, BERLIN DE Seiten 41 - 42 G. BOHEIM ET. AL. 'intrkardiale Impedanzmessung zur Regelung frequenzadaptiver Schrittmachersysteme'

## Beschreibung

Die Erfindung betrifft eine Anordnung zur Steuerung eines Herzschrittmachers der im Oberbegriff des Anspruchs 1 angegebenen Art.

Zur Steuerung der Stimulationsfrequenz eines Herzschrittmachers können verschiedene Meßgrößen herangezogen werden.

Auf der Grundlage neuerer Erkenntnisse werden bevorzugt physiologische Parameter, wie Temperatur des venösen Blutes, Atemfrequenz oder andere intrathorakale Funktionsparameter des Patienten, bei weitgehender Abstimmung aufeinander zur Steuerung der Stimulationsfrequenz benutzt. Ebenso ist über die Ermittlung des Schlagvolumens bzw. des Herzvolumens möglich, die Stimulationsfrequenz des Herzschrittmachers an bestimmte Belastungssituationen des Patienten anzupassen.

Aus der DE-A-36 29 587 oder US-A-4733667 ist eine Anordnung bekannt, mit der das Ventrikularvolumen in Verbindung mit einer Impedanzmessung ermittelt werden kann. Danach wird ein intrakavitärer Impedanzkatheter benutzt. Dieser Impedanzkatheter besteht aus einem intraventrikulär einzubringenden Rohr, das eine Mehrzahl von Paaren und im Abstand zueinander angeordneter Oberflächenelektroden aufweist. Diese Elektroden werden von einer entsprechenden Anzahl elektrischer Signale mit jeweils unterschiedlicher Frequenz angesteuert und dienen als Stromquelle bzw. Spannungsmeßpunkte. Das momentane Kammervolumen ist aus der gemessenen Spannung, der Größe des eingeprägten Stroms und einiger patientenspezifischer, geometrischer Größen rechnerisch ermittelbar. Mit steigender Anzahl der Elektrodenpaare laßt sich die Genauigkeit des Verfahrens erhöhen. Das Schlagvolumen ergibt sich aus der Differenz des berechneten Minimalvolumens für die Endsystole und dem Maximalvolumen für die Enddiasstole.

Der vorgeschlagene vielpolige Impedanzkatheter ist zwar zur Bestimmung des Herzkammervolumens grundsätzlich geeignet, er besitzt jedoch für eine Anwendung in Verbindung mit dauerhaft implantierten Herzschrittmachern folgende den wesentlichen Nachteil, daß die erforderliche größere Anzahl parallel verlaufender und voneinander isoliert angeordneten Zuleitungsdrähte mit erheblichem konstruktiven Aufwand verbunden ist, wenn bei den, durch den permanenten Einsatz erforderlichen Biegebelastungen des Impedanzkatheters eine sichere Kontaktierung der entsprechenden Elektroden gewährleistet werden soll. Als weiterer Nachteil kommt hinzu, daß die erforderliche vielpolige Steckverbindung zum Herzschrittmacher zu einer erheblichen Volumenvergrößervng führt. Desweiteren ist es bei der vorgeschlagenen Meßmethode nicht möglich, bereits implantierte Elektrodenanordnungen für eine Impedanzmessung heranzuziehen, so daß die zusätzlichen Steckverbindungen das Volumen des Schrittmachers noch weiter erhöhen.

Aus US-A-4702253 ist es bekannt zur Impedanzmessung Elektroden von herkömmlichen Schrittmacherleitungen einzusetzen, jedoch wird hier das Vorsehen zusätzlicher Elektroden eigens für die Impedanzmessung vorgeschlagen, und die Möglichkeiten der herkömmlichen Leitungen für diesen Zweck werden nicht ausgeschöpft.

Der Erfindung liegt die Aufgabe zugrunde, eine Anordnung zur Steuerung eines Herzschrittmachers der eingangs genannten Gattung anzugeben, bei der konventionelle Elektrodensysteme, die in Verbindung mit Zweikammer-Herzschrittmachern bereits erfolgreich eingesetzt worden sind, zur Gewinnung eines mit dem Herzvolumen korrelierenden Signals bei gleichzeitig ausreichender Meßgenauigkeit herangezogen werden können.

Diese Aufgabe wird mit den kennzeichnenden Merkmalen des Anspruchs gelöst.

Die Erfindung schließt die Erkenntnis ein, daß es für eine präzise Steuerung der Stimulationsfrequenz des Herzschrittmachers ausreichend ist, die Messung des absoluten Volumens der Herzkammer durch eine relative Volumenmessung zu ersetzen. Eine derartige Vorgehensweise ist dadurch gerechtfertigt, daß zur Anpassung der Stimulationsfrequenz nicht die exakte Kurvenform des Volumensignals, sondern nur die Veränderung relativer Werte zu definiert vorgegebenen Herzphasen erfaßt werden muß. Eine, wie auch immer geartete, im wesentlichen jedoch eindeutige Funktionalbeziehung zwischen der gemessenen Impedanz und dem tatsächlichen Volumen ist hinreichend.

Die Positionierung der erfindungsgemäß benutzten intrakardialen Sonden in einem Atrium und in einem Ventrikel des Herzens eines Patienten ermöglicht die Impedanzmessung über ein relativ großes Volumen und führt dadurch in vorteilhafter Weise zu einer hohen Meßgenauigkeit für das relative Herzvolumen, dessen aus der Impedanz-Meßgröße rechnerisch ermittelter Wert für die Anpassung der Stimulationsfrequenz des Herzschrittmachers herangezogen wird. Der Einschluß des zwischen Atrium und Ventrikel befindlichen Herzklappensystems in dem zur Messung der Impedanz benutzten Blutvolumen beeinflußt die Meßgenauigkeit des Verfahrens nur unwesentlich, da aufgrund der Bestimmung des relativen Kammervolumens mögliche Fehlerquellen eliminiert werden.

Nach einer bevorzugten Ausführungsform der Erfindung werden implantierte atriale und ventrikuläre bipolare Sonden bekannter Bauart außer zur Stimulation des Patientenherzens zusätzlich zur Impedanzmessung genutzt. Damit entfällt die Verlegung zusätzlicher Elektroden.

Die insbesondere ringförmig ausgestalteten Elektroden der atrialen und der ventrikulären bipolaren Sonde werden dabei außerhalb der Stimulationszeitpunkte zur Einspeisung eines Stroms aus einer Konstant-Stromquelle in das Meßvolumen benutzt. Zwischen den Ringelektroden baut sich aufgrund des eingeprägten Stroms ein elektrisches Feld auf, dessen Potentialgefälle über die Spitzenelektroden der atrialen und ventrikulären bipolaren Sonden als eine dem Kammervolumen proportionale Meßspannung abgegriffen werden kann. Die Spitzenelektroden der beiden bipolaren Sonden bilden dabei ein erstes und die Ringelektroden ein zweites Elektrodenpaar.

Die Zuordnung der einzelnen Elektroden der atrialen bzw. ventrikulären Sonden erfolgt bevorzugt über geeignete Schaltmittel und deren Steuerglieder, die in Abhängigkeit des patientenabhängigen Zeittaktes des Herzschrittmachers über dessen zentrale Steuerungs- und Signalverarbeitungs-Einheit den jeweils aktiven Signalquellen und -senken verfügbar gemacht werden.

Nach einer günstigen Weiterbildung der Erfindung sind die Schaltmittel als bipolare Umschalter ausgebildet. Sie werden jeweils paarweise der im Atrium bzw. im Ventrikel vorgesehenen Sonde zugeordnet. Dadurch ist es auf einfache Weise möglich, die erforderliche Zuordnung der Meßsignale und Stimulierungs- bzw. Kontrollimpulse zwischen den intrakardialen Elektroden und der zentralen Steuerungs- und Signalverarbeitungs-Einheit sowie einer die Konstant-Stromquelle und einen Spannungsmesser aufweisenden Impedanzmeß-Einheit herzustellen. Die Umschalter werden immer dann betätigt, wenn weder ein Stimulationsimpuls noch ein Sensing-Signal auf den Verbindungsleitungen zwischen den intrakardialen Elektroden und der zentralen Steuerungs- und Signalverarbeitungs-Einheit des Herzschrittmachers vorhanden ist. Für diese Aufgabe sind als Steuerglieder der Schaltmittel handelsübliche NOR-Elemente mit besonderem Vorteil einsetzbar.

Gemäß einer anderen vorteilhaften Weiterbildung der Erfindung ist im Ventrikel eine unipolare Sonde angeordnet. Um einen störfreien Ablauf der Meß- und Stimulierungsaufgaben des Herzschrittmachers zu sichern, ist die vierte Elektrode durch das Gehäuse des Schrittmachers gebildet.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
- Figur 1: die Anordnung der intrakardialen Sonden nach der bevorzugten Ausführungsform der Erfindung in schematisierter Darstellung,
- Figur 2: das Blockschaltbild einer bevorzugten Ausführungsform einer Vorrichtung zur Verfahrensdurchführung in schematisierter Form
und
- Figur 3: das schematisiert dargestellte Blockschaltbild einer günstigen Weiterbildung der in Figur 2 gezeigten Vorrichtung.

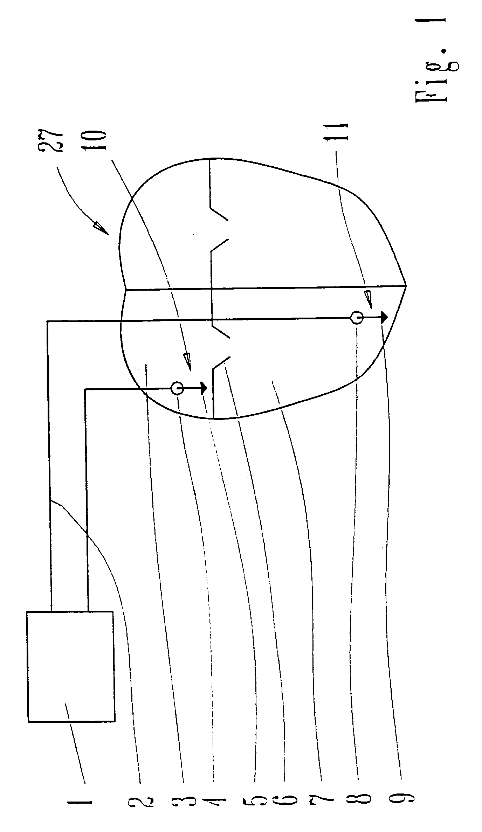

Die schematisierte Darstellung gemäß Figur 1 zeigt die Lage zweier zur Gewinnung eines mit dem Herzvolumen korrelierenden Signals verwendeter Sonden 10, 11 innerhalb des Herzens 27. Die Sonden 10, 11 sind bipolar ausgebildet und besitzen jeweils eine Spitzenelektrode 5, 9 und eine Ringelektrode 4, 8. Die durch Leitungen 2 mit dem Herzschrittmacher 1 verbundenen Sonden 10, 11 befinden sich im Atrium 3 und im Ventrikel 7 des Herzens 27. Dabei bilden die atriale und die ventrikuläre Spitzenelektrode 5, 9 sowie die atriale und die ventrikuläre Ringelektrode 4, 8 der einzelnen Sonden ein erstes und ein zweites Elektrodenpaar. Das erste Elektrodenpaar dient der Erfassung der Spannung, die aufgrund einer Stromeinspeisung mittels des zweiten Elektrodenpaares 4, 8 und des daraus resultierenden Potentialunterschiedes über dem Blutwiderstand zwischen den Spitzenelektroden 5, 9 entsteht. Das momentane Kammervolumen läßt sich aus der durch die gemessene Spannung und den eingeprägten Strom bestimmten Impedanz und einigen geometrischen, herzspezifischen Größen ermitteln, um in Abhängigkeit davon die erforderliche Anpassung der Stimulationsfrequenz vornehmen zu können.

Für die Meßgenauigkeit des Verfahrens ist u. a. der relativ große räumliche Abstand der bipolaren atrialen und ventrikulären Sonden 10, 11 von besonderem Vorteil. Die Spitzenelektroden werden neben der Spannungsermittlung auch zur Anregung der Herzfunktion (Stimulierungs-Phase) und zur Erfassung der Herzreaktionen (Sensing-Phase) genutzt. Die Signalverarbeitung und die Impulserzeugung zur Stimulierung erfolgt durch geeignete Einrichtungen des Herzschrittmachers 1.

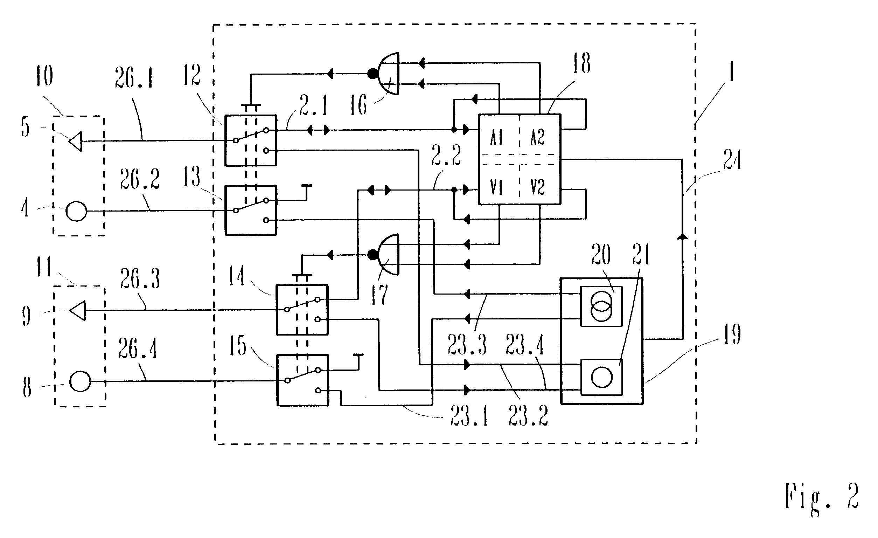

In Figur 2 ist der Aufbau einer bevorzugten, im Herzschrittmacher 1 angeordneten Vorrichtung zur erfindungsgemäßen Impedanzmessung in schematisierter Form dargestellt. Die Vorrichtung besitzt eine zentrale Steuerungs- und Signalverarbeitungs-Einheit 18, die vier Baugruppen A1, A2, V1 und V2 aufweist. Hier werden einerseits die Kontrollsignale (Sensing) für Eigenreaktion des Atriums 3 bzw. des Ventrikels 7 erfaßt und andererseits die Stimulationsimpulse in Abhängigkeit von der zu messenden Impedanz erzeugt. Zu diesem Zweck sind die Baugruppen A1, A2, V1 und V2 über die Leitungen 2.1 und 2.2 mit steuerbaren Schaltmitteln 12 und 14 verbunden, die ihrerseits über die Leitungen 26.1 bzw. 26.3 eine Verbindung mit den intrakardialen Spitzenelektroden 5 und 9 der bipolaren Sonden 10 und 11 aufweisen. Darüberhinaus besitzt die Vorrichtung eine Impedanzmeß-Einheit 19, die im wesentlichen in eine Konstantstrom-Quelle 20 und einen Spannungsmesser 21 untergliedert ist. Die Konstantstrom-Quelle 20 ist über die Leitungen 23.1 und 23.3, die steuerbaren Schaltmittel 13 und 15 sowie die Leitungen 26.2 und 26.4 mit den Ringelektroden 4 und 8 der bipolaren Sonden 10, 11 zwecks Einprägen eines konstanten Stroms in das Herzvolumen verbunden. Die zur Bestimmung des Herzvolumens erforderliche Impedanzmessung wird über die Ermittlung der durch den eingeprägten Konstantstrom hervorgerufenen Potentialunterschied realisiert. Dazu ist der Spannungsmesser 21 der Impedanzmeß-Einheit 19 über die Leitungen 23.2 und 23.4, die steuerbaren Schaltmittel 12 und 14 sowie dei Leitungen 26.1 und 26.3 ebenfalls mit den intrakardialen Spitzenelektroden 5, 9 verbindbar.

Um sowohl die Kontrolle und Steuerung der Herzfunktion als auch gleichzeitig die Impedanzmessung zur gezielten Anpassung der Stimulationsfrequenz durch den Herzschrittmacher 1 durchführen zu können, sind die Schaltmittel 12, 13, 14, 15 in Abhängigkeit von dem Arbeitszustand der zentralen Steuerungs- und Signalverarbeitungs-Einheit 18 steuerbar ausgebildet. Die als zweipolige umschalter ausgebildeten Schaltmittel sind den intrakardialen Sonden paarweise zugeordnet und werden durch die NOR-Bausteine 16 und 17 paarweise gesteuert. Der in Figur 2 dargestellte Zustand erfaßt den Zeitbereich, in dem die zentrale Steuerungs- und Signalverarbeitungs-Einheit 18 die Sensing-Signale vom Atrium bzw. Ventrikel empfängt oder Stimulierungs-Impulse an diese Herzabschnitte abgibt. Dabei ist die Impedanzmeß-Einheit 19 von der atrialen bzw. ventrikulären Sonde 10, 11 durch die Schaltmittel 12, 13 bzw. 14, 15 getrennt. Außerhalb dieses Zeitbereichs werden aufgrund des fehlenden Signalpegels an den Baugruppen A1, A2, V1 und V2 die NOR-Gatter 16, 17 umgeschaltet und ändern dadurch den Schaltzustand der Schaltmittel 12, 13, 14 und 15 dahingehend, daß die Verbindung der Sonden zu der zentralen Steuerungs- und Signalauswerte-Einheit 18 unterbrochen wird und die Konstant-Stromquelle 20 über die Schaltmittel 13 und 15 mit den Ringelektroden 4 und 8 der Sonden 10 und 11 und der Spannungsmesser 21 über die Schaltmittel 12 und 14 mit den Spitzenelektroden 5 und 9 der jeweiligen Sonde im Atrim 3 und im Ventrikel 7 verbunden ist. Die in diesem Schaltzustand der Schaltmittel 12 bis 15 gemessenen Impedanzwerte werden als ein mit dem augenblicklichen Herzvolumen korrelierendes Signal über die Leitung 24 der zentralen Steuerungs- und Signalverarbeitungs-Einheit 18 zwecks Steuerung der Stimulationsfrequenz zugeführt.

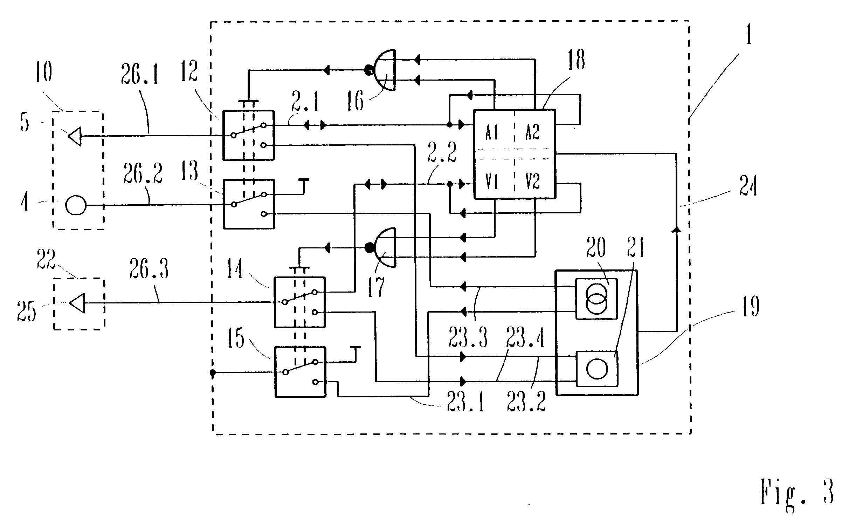

Eine Weiterbildung der Vorrichtung zur Impedanzmessung ist in Figur 3 in schematisierter Form dargestellt, bei der die bipolare ventrikuläre Sonde 11 durch eine unipolare Sonde 22 mit der Spitzenelektrode 25 ersetzt ist. Die fehlende vierte Elektrode wird durch das Potential des Gehäuses des Herzschrittmachers 1 ersetzt, so daß die Einspeisung des Stromes aus der Konstant-Stromquelle 20 über Schrittmachergehäuse und die Ringelektrode 4 der atrialen Sonde 10 vorgenommen werden kann.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei anders gearteten Ausführungen Gebrauch macht.

## Patentansprüche

1. Anordnung zur Steuerung eines Herzschrittmachers, bei dem ein mit dem Herzvolumen korrelierendes Signal durch eine Impedanzmessung unter Zuhilfnahme intrakardial angeordneter Elektroden erzeugt wird und eine Größe zur Beeinflussung der Stimulationsfrequenz bildet, wobei
eine das Herzvolumen repräsentierende Größe als relative Volumengröße in vorgegebenen Herzphasen aus einer zwischen einem ersten Paar von zwei einzelnen, örtlich voneinander getrennt angeordneten intrakardialen Elektroden (5, 9) gemessenen Impedanz ermittelt wird und diese Impedanz im wesentlichen aus dem Verhältnis des am ersten Elektrodenpaar (5, 9) anliegenden elektrischen Potentials und dem dieses Potential erzeugenden und über ein zweites Elektrodenpaar (4, 8) eingeprägten Konstantstrom bestimmt wird, **dadurch gekennzeichnet,** daß die Elektrodenpaare (5, 9 bzw. 4, 8) unter Nutzung einer atrial und einer ventrikulär positionierten Herzschrittmacher-Sonde (10, 11) gebildet werden, wobei zur Bildung der Elektrodenpaare eine atriale bipolare Sonde (10 oder 11) und eine ventrikuläre bipolare oder unipolare Sonde (22) vorgesehen sind und im Falle einer unipolaren ventrikulären Sonde die vierte erforderliche Elektrode durch das Gehäuse des Herzschrittmachers (1) gebildet wird.

2. Anordnung zur Steuerung eines Herzschrittmachers nach Anspruch 1, **dadurch gekennzeichnet,** daß die atriale und die ventrikuläre Sonde als bipolares System (10, 11) ausgebildet sind und beide Elektrodenpaare bilden.

3. Anordnung zur Steuerung eines Herzschrittmachers nach einem der vorangehenden Ansprüche, **dadurch gekennzeichnet,** daß zur Bildung der intrakardialen Elektrodenpaare die Ring- und/oder Spitzenelektroden (4, 5, 8, 9) der atrialen und ventrikulären Sonde (10, 11) vorgesehen sind.

4. Anordnung zur Steuerung eines Herzschrittmachers nach Anspruch 2, **dadurch gekennzeichnet,** daß die intrakardialen Elektroden (4, 5, 8, 9) der atrialen und/oder ventrikulären Sonde (10, 11, 22) sowohl die Sensing- und/oder Stimulationselektroden für den Herzschrittmacher (1) als auch Meßelektroden für eine Impedanzmeß-Einheit (19) bilden.

5. Anordnung nach einem der vorangehenden Ansprüche,
**gekennzeichnet durch**
- eine zentrale Steuerungs- und Signalverarbeitungs-Einheit (18), in welcher im Atrium (3) und im Ventrikel (7) des Herzens erzeugte Sensing-Impulse verarbeitet werden, von welcher Stimulationssignale abgegeben werden und die über Leitungen (2.1, 2.2, 26.1 und 26.3) mit den intrakardialen Elektroden (5, 9) der Sonden (10, 11, 22) im Atrium (3) bzw. im Ventrikel (7) verbunden ist;
- in den Leitungsverbindungen zwischen den Sonden (10, 11, 22) und der zentralen Steuerungs- und Signalverarbeitungs-Einheit (18) angeordnete Schaltmittel (12, 13, 14, 15),
- mit den Schaltmitteln (12, 13, 14, 15) verbundene und von der zentralen Steuerungs- und Signalverarbeitungs-Einheit (18) ansteuerbare Steuerglieder (16, 17) und
- eine Impedanzmeß-Einheit (19), welche über Leitungen (23.1, 23.2, 23.3, 23.4) mit den Schaltmitteln (12, 13, 14, 15) und über eine Leitung (24) mit der zentralen Steuerungs- und Signalverarbeitungs-Einheit (18) verbunden ist.

6. Anordnung nach Anspruch 7, **dadurch gekennzeichnet,** daß die Schaltmittel (12, 13, 14, 15) als zweipolige Umschalter ausgebildet sind.

7. Anordnung nach Anspruch 6, **dadurch gekennzeichnet,** daß die Schaltmittel (12, 13) gemeinsam schaltbar ausgebildet und der atrialen Sonde (10) zugeordnet sind.

8. Anordnung nach Anspruch 6, **dadurch gekennzeichnet,** daß die Schaltmittel (14, 15) gemeinsam schaltbar ausgebildet und der ventrikulären Sonde (11) zugeordnet sind.

9. Anordnung nach einem der Ansprüche 5 bis 8, **dadurch gekennzeichnet,** daß die Steuerglieder (16, 17) als NOR-Baustein ausgebildet sind.

10. Anordnung nach einem der Ansprüche 5 bis 9, **dadurch gekennzeichnet,** daß die Impedanzmeß-Einheit (19) eine Konstant-Stromquelle (20) und einen Spannungsmesser (21) aufweist.

11. Anordnung nach Anspruch 10, **dadurch gekennzeichnet,** daß die Konstant-Stromquelle (20) über die Leitungen (23.1, 23.3) mit der Ringelektrode (4, 8) der intrakardialen Sonden (10, 11) verbindbar ist.

12. Anordnung nach Anspruch 10, **dadurch gekennzeichnet,** daß die Konstant-Stromquelle (20) einerseits mit der Ringelektrode (4, 8) einer bipolaren Sonde (10, 11) und andererseits mit dem Gehäuse des Schrittmachers (1) verbindbar angeordnet ist.

13. Anordnung nach einem der Ansprüche 10 bis 12, **dadurch gekennzeichnet,** daß der Spannungsmesser (21) über Leitungen (23.2, 23.4) mit den Spitzenelektroden (5, 9) der bipolaren Sonden (10, 11) verbindbar angeordnet ist.

14. Anordnung nach einem der Ansprüche 10 bis 12, **dadurch gekennzeichnet,** daß der Spannungsmesser (21) über Leitungen (23.2, 23.4) mit der Spitzenelektrode (5, 9) einer bipolaren Sonde (10, 11) und der Spitzenelektrode (25) einer unipolaren Sonde (22) verbindbar angeordnet ist.

## Claims

1. Arrangement for controlling a heart pacemaker in which a signal correlated with the volume of the heart is generated by means of an impedance measurement with the aid of intracardially disposed electrodes, said signal constituting a quantity for affecting the stimulation frequency, wherein
a quantity representing the volume of the heart is established in the form of a relative volumetric capacity in predefined phases of the heart from an impedance measured between a first pair of two, individual, spatially separated intracardially disposed electrodes (5, 9), said impedance being substantially determined by the ratio of the electric potential present on the first pair of electrodes (5, 9) and the constant current producing this potential that is impressed via a second pair of electrodes (4, 8), characterized in that the pairs of electrodes (5, 9 and 4, 8) are formed by utilising a respective heart pacemaker probe (10, 11) positioned in the atrium and in the ventricle whereby an atrial bipolar probe (10 or 11) and a ventricular bipolar or unipolar probe (22) are provided for forming the pairs of electrodes and, in the case of a unipolar ventricular probe, the necessary fourth electrode is formed by the housing of the heart pacemaker (1).

2. Arrangement for controlling a heart pacemaker in accordance with Claim 1, characterized in that the atrial and the ventricular probes are constructed in the form of a bipolar system (10, 11) and form the two pairs of electrodes.

3. Arrangement for controlling a heart pacemaker in accordance with any of the preceding Claims, characterised in that the annular and/or point electrodes (4, 5, 8, 9) of the atrial and ventricular probes (10, 11) are used for forming the intracardial pairs of electrodes.

4. Arrangement for controlling a heart pacemaker in accordance with Claim 2, characterised in that the intracardial electrodes (4, 5, 8, 9) of the atrial and/or ventricular probes (10, 11, 22) form the sensing and/or stimulation electrodes for the heart pacemaker (1) and also act as test electrodes for an impedance measuring unit (19).

5. Arrangement in accordance with any of the preceding Claims, characterised by
- a central control and signal processing unit (18) in which the sensing pulses produced in the atrium (3) and in the ventricle (7) of the heart are processed and from which said unit the stimulation signals are emitted, said unit being connected over leads (2.1, 2.2, 26.1 and 26.3) to the intracardial electrodes (5, 9) of the respective probes (10, 11, 22) in the atrium (3) and in the ventricle (7);
- switching means (12, 13, 14, 15) disposed in the connecting leads between the probes (10, 11, 22) and the central control and signal processing unit (18),
- control members (16, 17) connected to the switching means (12, 13, 14, 15) and controllable by the central control and signal processing unit (18), and
- an impedance measuring unit (19) which is connected over leads (23.1, 23.2, 23.3, 24.4) to the switching means (12, 13, 14, 15) and over a lead (24) to the central control and signal processing unit (18).

6. An arrangement in accordance with Claim 7 [sic], characterised in that the switching means (12, 13, 14, 15) take the form of two-pole changeover switches.

7. Arrangement in accordance with claim 6, characterised in that the switching means (12, 13) are ganged and are associated with the atrial probe (10).

8. Arrangement in accordance with Claim 6, characterised in that the switching means (14, 15) are ganged and are associated with the ventricular probe (11).

9. Arrangement in accordance with any of the Claims 5 to 8, characterised in that the control members (16, 17) take the form of NOR modules.

10. Arrangement in accordance with any of the Claims 5 to 9, characterised in that the impedance measuring unit (19) comprises a constant current source (20) and a voltmeter (21).

11. Arrangement in accordance with Claim 10, characterised in that the constant current source (20) is connectable via the leads (23.1, 23. 3) to the annular electrodes (4, 8) of the intracardial probes (10, 11).

12. Arrangement in accordance with Claim 10, characterised in that the constant current source (20) is arranged so as to be connectible to the annular electrodes (4, 8) of a bipolar probe (10, 11) on the one hand and to the housing of the pacemaker (1) on the other.

13. Arrangement in accordance with any of the Claims 10 to 12, characterised in that the voltmeter (21) is arranged so as to be connectible to the point electrodes (5, 9) of the bipolar probes (10, 11) via the leads (23.2, 23.4).

14. Arrangement in accordance with any of the Claims 10 to 12, characterised in that the voltmeter (21) is arranged so as to be connectible to the point electrode (5, 9) of a bipolar probe (10, 11) and to the point electrode (25) of a unipolar probe (10) via the leads (23.2, 23.4)

## Revendications

1. Dispositif de commande d'un stimulateur cardiaque, dans le cas duquel un signal corrélé avec le volume cardiaque est produit par une mesure d'impédance à l'aide d'électrodes intracardiaqucs implantées et forme une valeur pour influencer la fréquence de stimulation, étant précisé qu'une valeur représentant le volume cardiaque est déterminée, en tant que valeur relative du volume, dans des phases prescrites du coeur, à partir d'une impédance mesurée entre une première paire de deux électrodes intracardiaques particulières, disposées séparées spatialement l'une de l'autre, et que cette impédance est essentiellement déterminée à partir du rapport entre le potentiel électrique existant à la première paire d'électrodes (5, 9) et le courant constant produisant ce potentiel et amené par l'intermédiaire d'une seconde paire d'électrodes (4,8), caractérisé par le fait que les paires d'électrodes (5, 9 ou 4, 8) sont formées par utilisation d'une sonde (10, 11) du stimulateur cardiaque implantés dans l'oreillette et dans le ventricule, étant précisé que, pour former les paires d'électrodes sont prévues une sonde bipolaire (10 ou 11) implantée dans l'oreillette et une sonde bipolaire ou unipolaire (22) implantée dans le ventricule et que, dans le cas d'une sonde ventriculaire unipolaire, la quatrième électrode nécessaire est formée par le boîtier des stimulateurs cardiaques (1).

2. Dispositif de commande d'un stimulateur cardiaque selon la revendication 1, caractérisé par le fait que la sonde implantée dans l'oreillette et la sonde implantée dans le ventricule sont conçues sous forme de systèmes bipolaires (10, 11) et qu'elles forment les deux paires d'électrodes.

3. Dispositif de commande d'un stimulateur cardiaque selon l'une des revendications précédentes, caractérisé par le fait que pour former les paires d'électrodes intracardiaques sont prévues des électrodes annulaires et/ou à pointes (4, 5, 8, 8, 9) de la sonde implantée dans l'oreillette et de celle implantée dans le ventricule (10, 11).

4. Dispositif de commande d'un stimulateur cardiaque selon la revendication 2, caractérisé par le fait que les électrodes intracardiaques (4, 5, 8, 9) de la sonde implantée dans l'oreillette et/ou de celle implantée dans le ventricule (10, 11, 22) forment aussi bien les électrodes de détection et/ou de stimulation pour le stimulateur cardiaque (1) qu'également des électrodes de mesure pour un organe (19) de mesure d'impédance.

5. Dispositif selon l'une des revendications précédentes, caractérisé par
- un organe central (10) de commande et de traitement du signal, dans lequel sont traitées des impulsions de détection qui sont produites dans l'oreillette (3) et dans le ventricule (7) du coeur et desquelles sont dérivés des signaux de stimulation et qui est relié, par des conducteurs (2.1, 2.2, 26.1; 26.3), avec les électrodes intracardiaques (5, 9) des sondes (10, 11, 22) implantées dans l'oreillette (3) ou dans le ventricule (7);
- par des commutateurs (12, 13, 14, 15) disposés sur les liaisons de conducteurs entre les sondes (10, 11, 22) et l'organe central (18) de commande et de traitement du signal,
- par des éléments de commande (16, 17) qui sont reliés aux commutateurs (12, 13, 14, 15) et peuvent être commandés par l'organe central (18) de commande et de traitement du signal et
- par un organe (19) de mesure de l'impédance qui est relié, par l'intermédiaire de conducteurs (23.1, 23.2, 23.3, 23.4), avec les commutateurs (12, 13, 14, 15) et, par l'intermédiaire d'un conducteur (24), avec l'organe central (18) de commande et de traitement du signal.

6. Dispositif selon la revendication 1, caractérisé par le fait que les commutateurs (12, 13, 14, 15) sont conçus sous forme de commutateurs bipolaires.

7. Dispositif selon la revendication 6, caractérisé par le fait que les commutateurs (12, 13) sont conçus commutables en commun et correspondent à la sonde (10) implantée dans l'oreillette.

8. Dispositif selon la revendication 6, caractérisé par le fait que les commutateurs (14, 15) sont conçus commutables en commun et correspondent à la sonde ventriculaire (11).

9. Dispositif selon l'une des revendications 5 à 8, caractérisé par le fait que les éléments de commande (16, 17) sont conçus sous forme d'éléments OU inversé.

10. Dispositif selon l'une des revendications 5 à 9, caractérisé par le fait que l'organe (19) de mesure d'impédance présente une source de courant constant (20) et un appareil de mesure de la tension (21).

11. Dispositif selon la revendication 10, caractérisé par le fait que la source de courant constant (20) peut être reliée, par l'intermédiaire des conducteurs (23.1, 23.3), avec les électrodes annulaires (4, 8) des sondes intracardiaques (10, 11).

12. Dispositif selon la revendication 10, caractérisé par le fait que la source de courant constant (20) est disposée pour être reliée d'une part avec l'électrode annulaire (4, 8) d'une sonde bipolaire (10, 11) et d'autre part avec le boîtier du stimulateur cardiaque (1).

13. Dispositif selon l'une des revendications 10 à 12, caractérisé par le fait que l'appareil (21) de mesure de la tension est disposé pour être relié, par l'intermédiaire de conducteurs (23.2, 23.4), avec les électrodes à pointes (5, 9) des formes bipolaires (10, 11).

14. Dispositif selon l'une des revendications 10 à 12, caractérisé par le fait que l'appareil (21) de mesure de la tension est disposé pour être relié, par l'intermédiaire de conducteurs (23.2, 23.4), avec l'électrode à pointes (5, 9) d'une sonde bipolaire (10, 11) et avec l'électrode à pointes (25) d'une sonde unipolaire (22).
